# EUROPEAN PATENT SPECIFICATION

(11) **EP 0 688 901 B1**
(45) Date of publication and mention of the grant of the patent: **06.10.1999**
(21) Application number: 95109536.3
(22) Date of filing: 20.06.1995
(51) Int. Cl.: D21H 21/22, D21H 17/06, D21H 17/07, D21H 17/13, A61K 7/48

(54) **Tissue paper containing glycerin and quaternary ammonium compounds**
Glycerin und quaternäre Ammonium-Verbindungen enthaltendes Tissuepapier
Papier tissu contenant de la glycérine et des composés d'ammonium quaternaire

(30) Priority: 21.06.1994 US 263109; 08.03.1995 US 400896
(43) Date of publication of application: 27.12.1995
(73) Proprietor: KIMBERLY-CLARK WORLDWIDE, INC., Neenah, Wisconsin 54956 (US)
(72) Inventor: Funk, Barbara Sue, Weyauwega, Wisconsin 54983 (US); Kryzsik, Duane Gerard, Appleton, Wisconsin 54911 (US); Pazdernik, Patrick Alan, Neenah, Wisconsin 54956 (US)
(74) Representative: DIEHL GLAESER HILTL & PARTNER

(56) References cited:
- GB-A- 2 121 449
- US-A- 5 240 562
- US-A- 5 279 767

## Description

The present invention relates to a tissue containing glycerin and quarternary ammonium compounds.

In the field of tissue development and production, considerable efforts have been directed toward improving the softness of the tissue. This has been approached in a variety of ways, generally by either improving the tissue basesheet or by adding chemicals to the tissue to provide improved feel. The addition of mineral oil or polysiloxanes, for example, are chemicals which provide a more smooth feel to the surface of the tissue. While the feel of the tissue is an important characteristic, the use of tissues offer an opportunity to provide other benefits to the user.

Documents US-A-5.240,562 and US-A-5.279,767 describe chemical softening compositions useful for the softening of paper products. The disclored softening compositions comprise quarternary ammonium compounds and a polyhydroxy compound. The softening composition is added to the aqueous slurry of the papermaking fibers or added subsequent to the formation of a wet tissue web and prior to drying of the web to completion. Document GB-A-2121449 describes compositions for the use in the manufacture of paper which comprise an imidazolinium dicarboxylate derivative, an alcohol, and a quaternary ammonium compound. The disclosed softening composition can be added to the stock for forming the web or can be sprayed onto the web prior to drying.

It is the object of the present invention to provide a soothing tissue having improved properties. This object is solved by the tissue of independent claim 1. Further advantageous features, aspects and details of the invention are evident from the dependent claims and the description.

It has now been discovered that a superior soft tissue can provide a soothing feel by incorporating into the tissue an aqueous softening compositioon containing a combination of selected ingredients by spraying or printing said softening composition on the surface of the through dried tissue. In general, the invention resides in a tissue to which has been added an aqueous composition comprising glycerin and one or more particular organic quaternary ammonium compounds.

More specifically, the quaternary ammonium compound(s) is/are selected from the group consisting of the following quaternary classes:
monoalkyl trimethyl quaternary amines having the following structure: 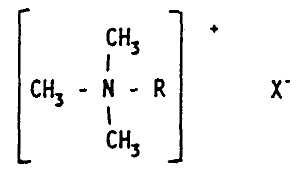 wherein X = chloride or methyl sulfate and R = aliphatic, saturated or unsaturated C₁₂ - C₂₂;
benzyl quaternary amines having the following structure: 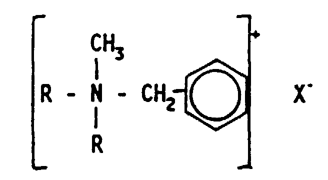 wherein X = chloride and R = aliphatic, normal C₁₂ - C₁₈;
benzyl quaternary amines, such as stearalkonium chloride, having the following structure: 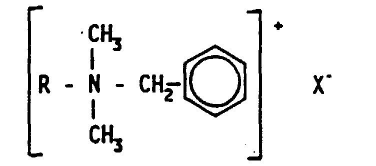 wherein X = chloride and R = straight chain C₁₈;
monomethyl trialkyl quaternary amines having the following structure: 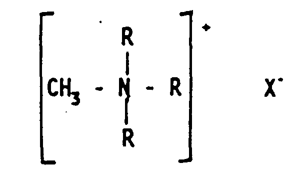 wherein X = chloride and R = aliphatic alkyl, normal or branched, C₈ - C₁₈;
imidazolinium quaternary amines having the following structure: 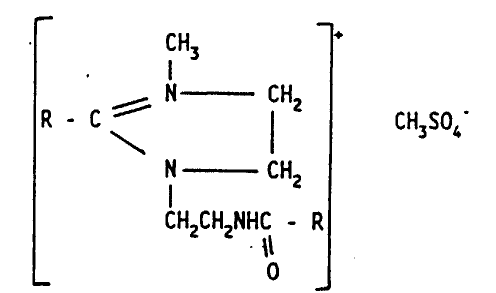 wherein R = aliphatic, normal, saturated or unsaturated, C₁₂ - C₁₈;
silicone quaternary amines having the following structure: 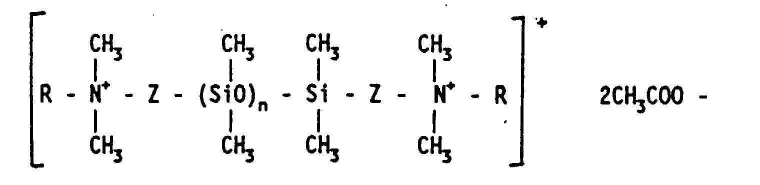 wherein Z = 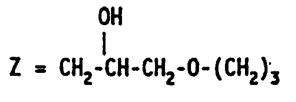 and R = long chain alkyl group, C₁₂ - C₁₈; and
quaternized lanolin derivatives, such as Quaternium-33, which have the following structure: 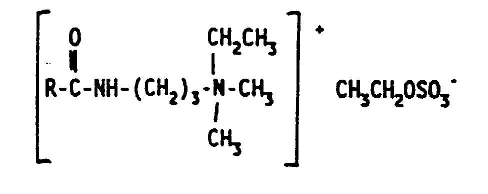 wherein RCO = lanolin acid radical.

The add-on amount of the softening composition is from about 3 to about 30 dry weight percent based on the weight of the tissue, more specifically from about 3 to about 20 dry weight percent, and still more specifically from about 5 to about 15 dry weight percent. The higher add-on amounts are more likely to leave behind a detectable residue on the skin, whereas the lower add-on amounts are less likely to do so. Water can be added to the formulation to reduce the viscosity of the glycerin and to make the formulation more suitable for application.

The amount of the quaternary ammonium compound in the aqueous softening composition can be from about 0.2 to about S weight percent, more specifically from about 0.3 to about 3 weight percent, and still more specifically from about 0.5 to about 1 weight percent.

The amount of glycerin in the aqueous softening composition is from about 20 to about 98 weight percent, more specifically from about 60 to about 80 weight percent, and still more specifically from about 40 to about 60 weight percent.

In addition, the softening composition can contain from about 0.5 to about 50 weight percent propylene glycol, more specifically from about 5 to about 30 weight percent propylene glycol. The propylene glycol can be used as a partial substitute for the glycerin in such formulations.

Also, the softening composition can contain from about 0.5 to about 50 weight percent polyethylene glycol, more specifically from about 5 to about 30 weight percent. The polyethylene glycol preferably has a molecular weight in the range of from about 200 to about 750. The polyethylene glycol can be used as a partial substitute for glycerin or propylene glycol in the softening composition.

Other optional ingredients include aloe, humectants, skin protectants, preservatives, and feel modifiers. Suitable humectants include lactic acid and its salts, sugars, ethoxylated glycerin, ethoxylated lanolin, corn syrup, hydrolyzed starch hydrolysate, urea, and sorbitol. Suitable skin protectants include allantoin, kaolin, and zinc oxide. Suitable feel modifiers include corn starch, oat flour, talc, boron nitride, and cyclodextrin.

The softening composition, which can be in the form of an aqueous solution or suspension, is incorporated into the tissue by spraying or printing onto the surface of the through dried tissue.

The tissue to which the softening composition is applied can be any tissue. Especially useful is the addition to facial tissue, bath tissue, or towels. Such tissues are produced by throughdrying tissue making processes and can be creped or uncreped, layered or non-layered (blended).

### Examples

### Example 1 (comparative)

A solution consisting of 80 parts by weight glycerin and 19 parts by weight deionized water and 1 part aloe was prepared by mixing the three ingredients until uniform. The resulting solution was then applied to a three-ply, wet-pressed, creped tissue having a basis weight of about 45 grams per square meter using a spray apparatus. The add-on amounts included 5, 8, and 10 dry weight percent based on the weight of the tissue. The resulting tissue samples did not provide any unusual benefit.

### Example 2 (comparative)

A softening composition consisting of 70 parts by weight glycerin, 19 parts by weight deionized water, 1 part by weight of Lanoquat 1751-A (a blend of Quaternium-33 (quaternized lanolin) and propylene glycol sold by Henkel Corporation, Ambler, PA) and 1 part by weight of aloe vera. The glycerin and Lanoquat 1751-A were mixed together first until uniform. Then the water and aloe vera was added and the mixture stirred until a homogeneous solution was achieved. The resulting softening solution was applied to a three-ply, wet-pressed, creped tissue having a basis weight of about 45 grams per square meter using a spray apparatus. The add-on amounts included about 10 and about 12 dry weight percent based on the dry weight of the tissue. The resulting tissue samples were unusually soft at both add-on levels.

### Example 3

A softening composition was prepared consisting of 60 parts by weight glycerin, 20 parts by weight propylene glycol, 1 part by weight Lanoquat 1751-A, 19 parts by weight deionized water, and 1 part by weight aloe vera. The glycerin and propylene glycol were mixed together until uniform. The Lanoquat 1751-A was added and mixed until uniform. The water and aloe vera was then added and the solution was stirred until homogenous. The resulting softening composition was applied to a two-ply throughdried tissue having a basis weight of about 42 grams per square meter using a spray apparatus. The add-on amounts included 6, 9, and 12 dry weight percent based on the weight of the tissue. In addition, the resulting softening composition was applied to a four-ply, wet-pressed, creped tissue having a basis weight of about 45 grams per square meter using a spray apparatus. The add-on amounts included 6, 9, and 12 dry weight percent based on the weight of the tissue.

### Example 4

A softening composition was prepared consisting of 60 parts by weight glycerin, 20 parts by weight propylene glycol, 5 parts by weight Lanoquat 1751-A, and 14 parts by weight deionized water, and 1 part by weight aloe vera. The glycerin and propylene glycol were mixed together until uniform. The Lanoquat 1751-A was added and mixed until uniform. The water and aloe vera was then added and the solution was stirred until homogeneous. The resulting softening composition was applied to a two-ply, throughdried creped tissue having a basis weight of about 42 grams per square meter using a spray apparatus. The add-on amounts included 6, 9, and 12 dry weight percent based on the weight of the tissue.

A sensory panel evaluated tissue samples from Example 3 and Example 4 versus an untreated two-ply, throughdried tissue, and a two-ply, throughdried tissue treated with a silicone at an add-on of 3 dry weight percent based on the weight of the tissue. The samples from Examples 3 and 4 were as good as or better for softness than the silicone-treated sample and better than the untreated control for softness. Example 4 with a 12 percent add-on had the best softness of all the samples, followed by Example 3 with a 12 percent add-on.

### Example 5

A softening composition was prepared consisting of 40 parts by weight of propylene glycol, 1 part by weight of stearalkonium chloride, and 19 parts by weight of deionized water. The propylene glycol and the stearalkonium chloride were mixed together and heating until uniform. The glycerin was added and mixed until uniform. The water was then added and the solution was stirred until homogenous. The resulting softening composition was applied to a two-ply, throughdried, creped tissue having a basis weight of about 27 grams per square meter using a spray apparatus. The add-on amount was about 10 dry weight percent based on the weight of the tissue.

The tissue of Example 5 was evaluated subjectively by a sensory panel and found to be softer, silkier, and smoother than the samples of Example 3.

In all examples, except Example 1, as the add-on level increased, the resulting tissue became noticeably softer. The lower add-on levels produced tissues which did not leave a noticeable residue on the skin.

## Claims

1. A tissue obtainable by spraying or printing on the surface of a through dried tissue from 3 to 30 dry weight percent of an aqueous softening composition, said softening composition comprising from 20 to 98 weight percent glycerin and from 0.2 to 5 weight percent of one or more quaternary ammonium compounds selected from the group consisting of:
monoalkyl trimethyl quaternary amines having the following structure: 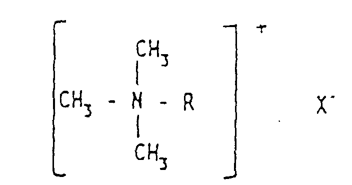 wherein X = chloride or methyl sulfate and R = aliphatic, saturated or unsaturated C₁₂ - C₂₂;
benzyl quaternary amines having the following structure: 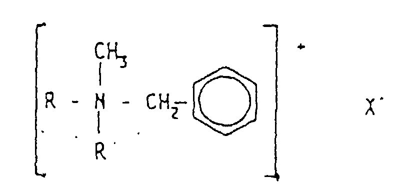 wherein X = chloride and R = aliphatic, normal C₁₂ - C₁₈;
monomethyl trialkyl quaternary amines having the following structure: 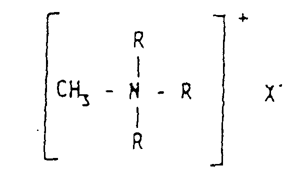 wherein X = chloride and R = aliphatic alkyl, normal or branched, C₈ - C₁₈;
imidazolinium quaternary amines having the following structure: 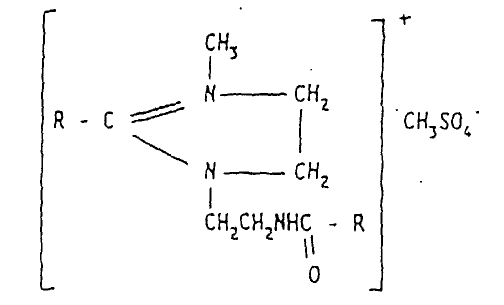 wherein R = aliphatic, normal, saturated or unsaturated, C₁₂ - C₁₈;
silicone quaternary amines having the following structure: 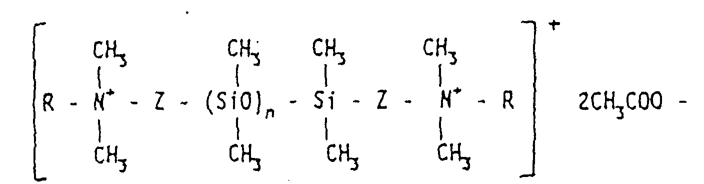 wherein 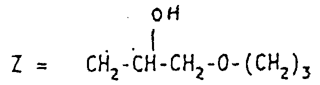 and R = long chain alkyl group, C₁₂ - C₁₈; and
quaternized lanolin derivatives which have the following structure: 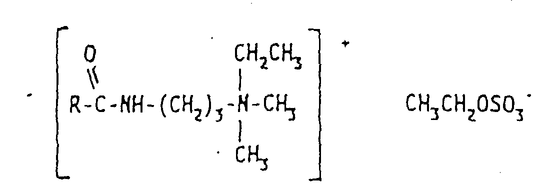 wherein RCO = lanolin acid radical.

2. The tissue of Claim 1 wherein the quaternary ammonium compound is a benzyl quaternary amine having the following structure: 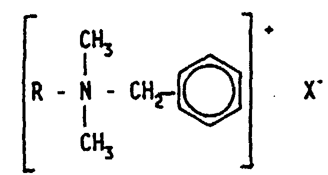 wherein X = chloride and R = straight chain C₁₈.

3. The tissue of Claim 1 wherein the quaternary ammonium compound is a silicone quaternary amine having the following structure: 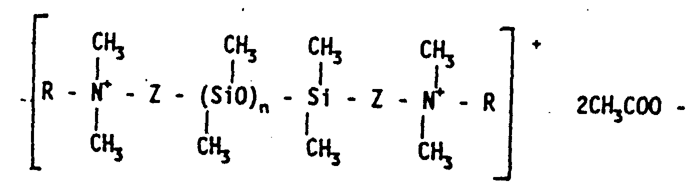 wherein Z = 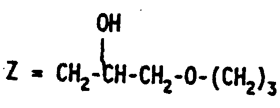 and R = long chain alkyl group, C₁₂ - C₁₈.

4. The tissue of one of the preceding claims, wherein the softening composition further comprises from 0.5 to 50 weight percent propylene glycol.

5. The tissue of one of the preceding claims, wherein the softening composition further comprises from 0.5 to 50 weight percent polyethylene glycol.

6. The tissue of one of the preceding claims, further comprising a humectant selected from the group consisting of lactic acid and its salts, sugars, ethoxylated glycerin, ethoxylated lanolin, corn syrup, hydrolyzed starch hydrolysate, urea, and sorbitol.

7. The tissue of one of the preceding claims, further comprising a skin protectant selected from the group consisting of allantoin, kaolin, zinc oxide, and dimethicone emulsions, talc, and starch.

8. The tissue of one of the preceding claims, further comprising a feel-modifier selected from the group consisting of com starch, oat flour, talc, boron nitride, and cyclodextrin.

9. The tissue of one of the preceding claims which is an absorbent tissue.

10. The tissue of one of the claims l to 8 which is a facial tissue.

11. The facial tissue of claim 10, comprising from 5 to 30 dry weight percent of a softening composition, said softening composition comprising from 60 to 80 weight percent glycerin, from 0.5 to 20 weight percent propylene glycol, and from 1 to 4 weight percent of a quaternized lanolin having the following structure:

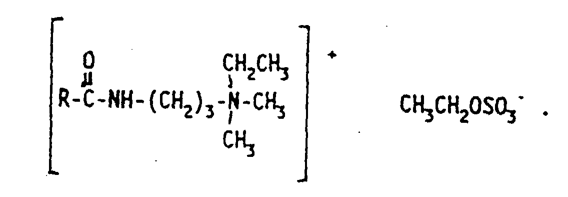

## Patentansprüche

1. Tissue, erhältlich durch Besprühen oder Bedrucken der Oberfläche eines durchgetrockneten Tissues mit 3 bis 30 Trockengewichtsprozent einer wäßrigen Weichmacherzusammensetzung, wobei die Weichmacherzusammensetzung 20 bis 98 Gew.% Glycerin und 0,2 bis 5 Gew.% einer oder mehrerer quaternärer Ammoniumverbindungen enthält, ausgewählt aus der Gruppe bestehend aus:
quaternären Monoalkyltrimethylaminen mit folgender Struktur: 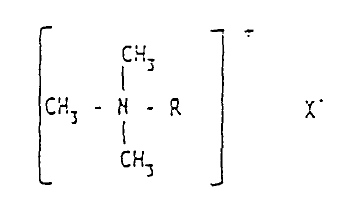 wobei X = Chlorid oder Methylsulfat und R = aliphatischer, gesättigter oder ungesättigter C₁₂-C₂₂-Rest;
quaternären Benzylaminen mit folgender Struktur: 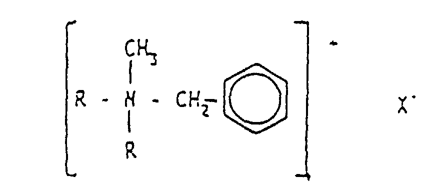 wobei X = Chlorid und R = aliphatischer, normaler C₁₂-C₁₈-Rest;
quaternären Monomethyltrialkylaminen mit folgender Struktur: 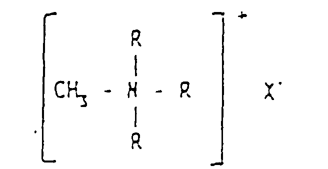 wobei X = Chlorid und R = aliphatischer, normaler oder verzweigter C₈-C₁₈-Alkylrest;
quaternären Imidazoliumaminen mit folgender Struktur: 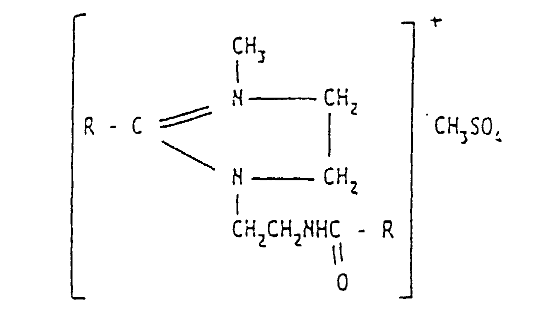 wobei R = aliphatischer, normaler, gesättigter oder ungesättigter C₁₂-C₁₈-Rest;
quaternären Silikonaminen mit folgender Struktur: 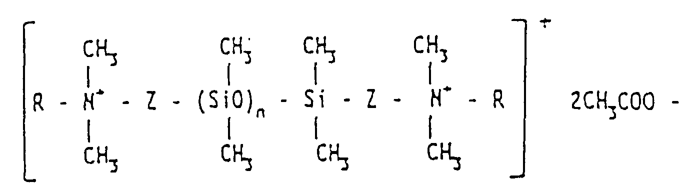 wobei 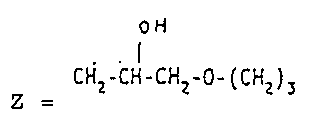 und R = langkettiger C₁₂-C₁₈-Alkylrest; und
quaternisierten Lanolinderivaten mit folgender Struktur: 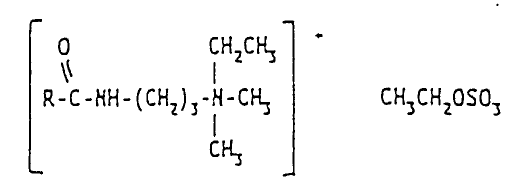 wobei RCO = Lanolinsäureradikal.

2. Tissue gemäß Anspruch 1, wobei die quaternäre Ammoniumverbindung ein quaternäres Benzylamin mit folgender Struktur ist: 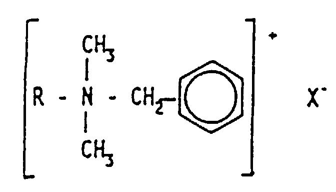 wobei X = Chlorid und R = geradkettiger C₁₈-Rest.

3. Tissue gemäß Anspruch 1, wobei die quaternäre Ammoniumverbindung ein quaternäres Silikonamin mit folgender Struktur ist: 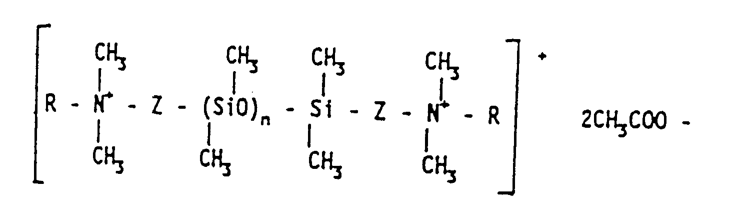 wobei 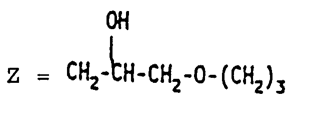 und R = langkettiger C₁₂-C₁₈-Alkylrest.

4. Tissue gemäß einem der vorhergehenden Ansprüche, wobei die Weichmacherzusammensetzung des weiteren 0,5 bis 50 Gew.% Propylenglycol enthält.

5. Tissue gemäß einem der vorhergehenden Ansprüche, wobei die Weichmacherzusammensetzung des weiteren 0,5 bis 50 Gew.% Polyethylenglycol enthält.

6. Tissue gemäß einem der vorhergehenden Ansprüche, mit des weiteren einem Benetzungsmittel ausgewählt aus der Gruppe bestehend aus Milchsäure und dessen Salzen, Zuckern, ethoxyliertem Glycerin, ethoxyliertem Lanolin, Stärkezuckersirup aus Mais, hydrolisiertem Stärkehydrolysat, Harnstoff und Sorbitol.

7. Tissue gemäß einem der vorhergehenden Ansprüche, mit des weiteren einem Hautschutzmittel ausgewählt aus der Gruppe bestehend aus Allantoin, Kaolin, Zinkoxid und Dimethiconemulsionen, Talk und Stärke.

8. Tissue gemäß einem der vorhergehenden Ansprüche, mit des weiteren einem Fühl-Modifikationsmittel ausgewählt aus der Gruppe bestehend aus Stärkezuckersirup aus Mais, Hafermehl, Talk, Bornitrid und Cyclodextrin.

9. Tissue gemäß einem der vorhergehenden Ansprüche, wobei es sich um ein saugfähiges Tissue handelt.

10. Tissue gemäß einem der Ansprüche 1 bis 8, wobei es sich um ein Gesichtstissue handelt.

11. Gesichtstissue gemäß Anspruch 10 mit 5 bis 30 Gew.% einer Weichmacherzusammensetzung, wobei die Weichmacherzusammensetzung 60 bis 80 Gew.% Glycerin, 0,5 bis 20 Gew.% Propylenglycol und 1 bis 4 Gew.% eines quaternisierten Lanolins mit folgender Struktur aufweist:

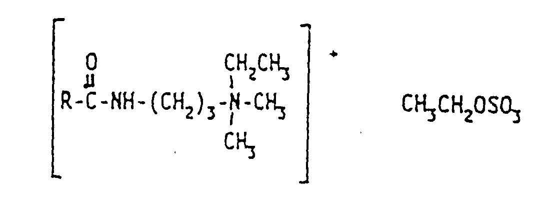

## Revendications

1. Papier-tissu mince pouvant être obtenu par pulvérisation ou impression, sur la surface d'un papier-tissu mince séché par soufflage transversal, de 3 à 30% en poids sec d'une composition assouplissante aqueuse, ladite composition assouplissante étant constituée de 20 à 98% en poids de glycérine et de 0,2 à 5% en poids d'un ou plusieurs composés ammonium quaternaire choisi dans le groupe consistant en :
- les monoalkyl-triméthylamines quaternaires ayant la structure suivante : 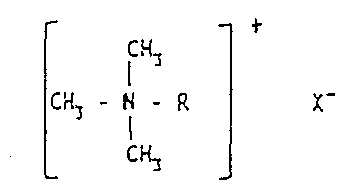 dans laquelle X = chlorure ou méthylsulfate et R = groupe aliphatique saturé ou insaturé en C₁₂-C₂₂ ;
- les benzylamines quaternaires ayant la structure suivante : 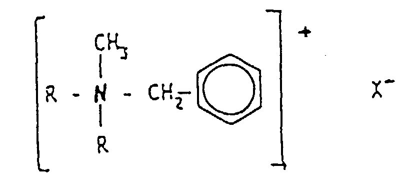 dans laquelle X = chlorure et R = groupe aliphatique linéaire en C₁₂-C₁₈ ;
- les monométhyl-trialkylamines quaternaires ayant la formule suivante 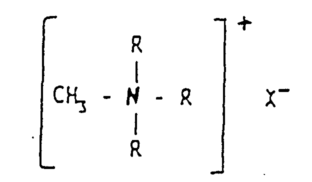 dans laquelle X = chlorure et R = groupe alkyle aliphatique, linéaire ou ramifié, en C₈-C₁₈ ;
- les imidazolinium amines quaternaires ayant la structure suivante : 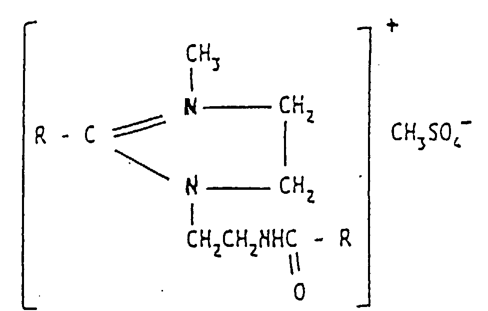 dans lesquelles R = groupe aliphatique linéaire, saturé ou insaturé, en C₁₂-C₁₈ :
- les silicone-amines quaternaires ayant la structure suivante : 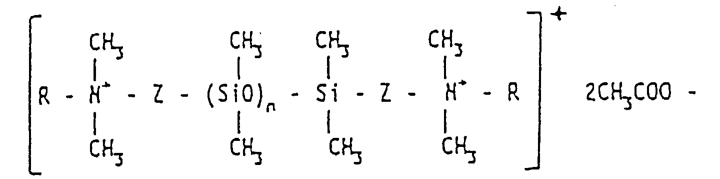 dans laquelle 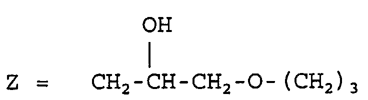 et R = groupe alkyle à chaîne longue en C₁₂-C₁₈ ; et
- les dérivés de la lanoline quaternisée ayant la structure suivante 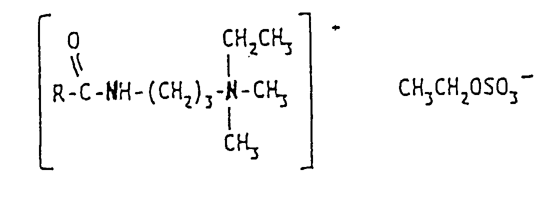 dans laquelle RCO = radical de l'acide de lanoline.

2. Papier-tissu mince selon la revendication 1, dans laquelle le composé ammonium quaternaire est une benzylamine quaternaire ayant la structure suivante : 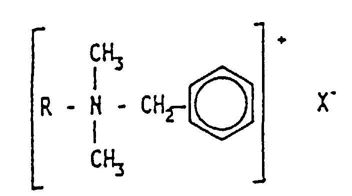 dans laquelle X = chlorure et R = groupe à chaîne droite en C₁₈.

3. Papier-tissu mince selon la revendication 1, dans lequel le composé ammonium quaternaire est une silicone-amine quaternaire ayant la structure suivante 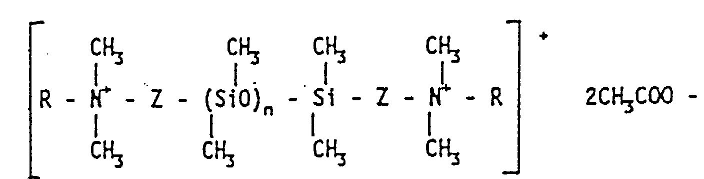 dans laquelle 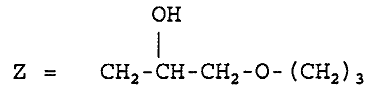 et R = groupe alkyle à chaîne longue en C₁₂-C₁₈.

4. Papier-tissu mince selon l'une des revendications précédentes, dans lequel la composition assouplissante comprend en outre de 0,5 à 50% en poids de propylène glycol.

5. Papier-tissu mince selon l'une des revendications précédentes, dans lequel la composition assouplissante comprend en outre de 0,5 à 50% en poids de polyéthylène glycol.

6. Papier-tissu mince selon l'une des revendications précédentes, comprenant en outre un humectant choisi dans le groupe consistant en l'acide lactique et ses sels, les sucres, la glycérine éthoxylée, la lanoline éthoxylée, le sirop de mais, l'hydrolysat d'amidon hydrolysé, l'urée et le sorbitol.

7. Papier-tissu mince selon l'une des revendications précédentes, comprenant en outre un agent protecteur de la peau choisi dans le groupe consistant en l'allantoine, le kaolin, l'oxyde de zinc et les émulsions de diméthicone, le talc et l'amidon.

8. Papier-tissu mince selon l'une des revendications précédentes, comprenant en outre un agent modificateur du toucher choisi dans le groupe consistant en l'amidon de mais, la farine d'avoine, le talc, le nitrure de bore et la cyclodextrine.

9. Papier-tissu mince selon l'une des revendications précédentes, qui est un papier-tissu mince absorbant.

10. Papier-tissu mince selon l'une des revendications 1 à 8, qui est un mouchoir.

11. Mouchoir selon la revendication 10 comprenant de 5 à 30% en poids sec d'une composition assouplissante, ladite composition assouplissante étant constituée de 60 à 80% en poids de glycérine, de 0,5 à 20% en poids de propylène glycol, et de 1 à 4% en poids de lanoline quaternisée ayant la structure suivante :
